Europäisches Patentamt

(19) European Patent Office

Office européen des brevets

(11) EP 0 681 448 B1

(12) FASCICULE DE BREVET EUROPEEN

(45) Date de publication et mention
de la délivrance du brevet:
07.01.1998 Bulletin 1998/02

(51) Int. Cl.$^6$: **A61B 5/024**, A61B 5/021,
A61B 5/022

(21) Numéro de dépôt: 94905746.7

(22) Date de dépôt: 27.01.1994

(86) Numéro de dépôt international:
PCT/FR94/00101

(87) Numéro de publication internationale:
WO 94/16616 (04.08.1994 Gazette 1994/18)

(54) **TENSIOMETRE A MESURE EN CONTINU, ET PROCEDE CORRESPONDANT**

VERFAHREN UND VORRICHTUNG ZUM KONTINUIERLICHEN MESSEN DES BLUTDRUCKS

METHOD AND DEVICE FOR CONTINUOUSLY MEASURING BLOOD PRESSURE

(84) Etats contractants désignés:
BE DE GB NL SE

(30) Priorité: 28.01.1993 FR 9301133

(43) Date de publication de la demande:
15.11.1995 Bulletin 1995/46

(73) Titulaire:
UNIVERSITE DE RENNES I
F-35000 Rennes (FR)

(72) Inventeurs:
• BILLON, Michel
F-22300 Lannion (FR)
• MARTIN, Eric
F-22700 Louannec (FR)
• CORAZZA, Michel
F-22700 Perros-Guirrec (FR)
• INGREMEAU, Olivier
F-22300 Lannion (FR)

(74) Mandataire: Vidon, Patrice
Cabinet Patrice Vidon,
Immeuble Germanium,
80, Avenue des Buttes-de-Coesmes
35700 Rennes (FR)

(56) Documents cités:
DE-A- 3 917 700          FR-A- 2 679 453
GB-A- 2 118 719          US-A- 4 409 983

## Description

Le domaine de l'invention est celui de la mesure de la tension artérielle d'un sujet, et plus généralement de toute information représentative du comportement du coeur d'un sujet. Plus précisément, l'invention concerne un dispositif permettant la mesure de la pression artérielle en continu, et éventuellement du rythme cardiaque (fonction pulsemètre).

La connaissance de la pression artérielle, et éventuellement du rythme cardiaque, intéresse de très nombreux utilisateurs. On peut notamment citer :

- les sportifs, et en particulier ceux participant à des épreuves de fond, au cours desquelles il est prudent de contrôler le comportement du coeur ;
- les personnes âgées sensibles à des problèmes cardiaques, qui doivent se soumettre à des contrôles fréquents pratiqués par un spécialiste, à domicile ou dans un centre spécialisé ;
- les personnes subissant des stress ;
- et plus généralement, toute personne souhaitant pouvoir obtenir à tout moment une information sur sa tension artérielle.

Dans le domaine médical, les appareils de mesure de tension (sphygmomètres) utilisent généralement le principe du garrot et nécessite un capteur (microphone) tenu par un brassard à serrage contrôlé placé autour du bras ou d'un doigt, ainsi qu'une boîte annexe de gestion et de stockage des mesures.

Le principe de la mesure consiste à desserrer progressivement le garrot. Lorsque le garrot est fortement serré, le capteur ne détecte aucun signal. Lorsqu'il atteint la pression systolique (SBP), un signal est détecté. Ce signal reste déctectable jusqu'à ce qu'on atteigne la pression diastolique (DBP). Ensuite, le capteur ne détecte plus rien. En d'autres termes, les valeurs SBP et DBP correspondent aux bornes de la zone dans laquelle un signal est détecté.

Les sphygmomètres présentent de nombreux inconvénients. Ils sont relativement encombrants et onéreux. Ils nécessitent une procédure d'utilisation complexe (serrage/desserrage) qui en limite l'utilisation au domaine médical. Par ailleurs, ils peuvent difficilement être portés en permanence par un sujet, à moins que celui-ci ne soit maintenu sur un lit d'hôpital.

Enfin, et principalement, les sphygmomètres ne délivrent qu'une information ponctuelle, et ne permettent pas de suivre aisément la tension artérielle. Ainsi, lorsqu'il est nécessaire de surveiller un sujet, on utilise des systèmes automatiques, qui contrôlent la mise en oeuvre d'un sphygmomètre à intervalles réguliers, et par exemple toutes les 10 minutes. Ces intervalles très longs peuvent entraîner une perte d'information, tel qu'un pic tensionnel passager.

On connaît également des dispositifs grand public permettant de mesurer la tension artérielle. Ainsi, la société CASIO (marque déposée) commercialise un tensiomètre, qui fonctionne suivant le principe de la mesure du temps de transit (PWTT) de l'onde de la pression sanguine.

Le temps de transit entre le départ de l'onde généré par le coeur est mesuré par la différence de potentiel électrique entre un point donné du corps qui peut être un doigt et le poignet de l'autre main. L'impulsion générée par la surpression cardiaque est détectée par mesure de la quantité d'hémoglobine à un instant donné. Un faisceau de lumière généré par une diode électroluminescente traverse un vaisseau sanguin puis un détecteur photosensible reçoit une fraction de la lumière réfléchie.

Ce tensiomètre est d'un usage très peu ergonomique. Il se présente sous la forme d'une montre portée à un poignet. Pour effectuer une mesure, il est nécessaire de placer un ou deux doigts de l'autre main sur des emplacements prévus à cet effet. Cette manipulation n'est pas si simple, et conduit, de même que les sphygmomètres, à une mesure ponctuelle de la tension.

L'invention a notamment pour objectif de pallier ces différents inconvénients de l'état de la technique.

On connaît encore des dispositifs intégrés dans une montre, et délivrant des données de pression sanguine, tels que décrits dans les documents GB 2 118 719 ou DE 3 917 700. Un capteur de pression est appliqué sur le corps du porteur, et une recherche des pics minimaux et maximaux du signal délivré par le capteur permet de délivrer des informations représentatives des pressions systolique et diastolique.

Ces dispositifs présentent les avantages d'être autonomes et de fonctionner en continu. Cependant, ils s'avèrent très imprécis, et ne peuvent pas être utilisés pour de nombreuses applications, notamment médicales. Ils peuvent en effet calculer l'écart entre la pression systolique et la pression diastolique, mais non fournir de façon précise leurs valeurs absolues, qui sont pourtant les plus significatives.

Plus précisément, un premier objectif de l'invention est de fournir un dispositif tensiomètre mesurant en permanence la tension artérielle d'un sujet.

L'invention a également pour objectif de fournir un tel tensiomètre qui permet de contrôler les évolutions de la tension du sujet.

Un autre objectif de l'invention est de fournir un tel tensiomètre, qui soit confortable et convivial pour l'utilisateur. En particulier, l'invention a pour objectif de fournir un tel tensiomètre, qui ne nécessite aucune manipulation ni aucune commande particulière. En d'autres termes, le tensiomètre de l'invention doit pouvoir fonctionner en continu de façon autonome. Par ailleurs, il doit être peu encombrant, et n'occasionner aucune gêne pour le porteur.

Un autre objectif de l'invention est encore de fournir un tel tensiomètre, qui soit d'un faible coût de revient, et qui soit aisément industrialisable.

Ces objectifs, ainsi que d'autres qui apparaîtront par la suite, sont atteints selon l'invention à l'aide d'un tensiomètre à mesure en continu de la tension artérielle d'un sujet, intégrant dans un boîtier portatif autonome :

- des moyens de mémorisation d'au moins une information de référence fonction de la tension artérielle dudit sujet dans un état prédéterminé,
- un capteur réagissant aux variations de la pression sanguine dudit sujet, délivrant un signal représentatif de ladite pression sanguine instantanée,
- des moyens de traitement dudit signal représentatif de la pression sanguine instantanée, délivrant au moins une information représentative des variations de tension artérielle dudit sujet, et
- des moyens de détermination de ladite tension artérielle dudit sujet, en fonction de ladite ou desdites informations de référence et de ladite et ou desdites informations représentatives des variations de tension artérielle.

Ainsi, selon l'invention la tension artérielle peut être connue en permanence (ou à intervalles réguliers), sans aucune manipulation. La mesure effectuée est relative, mais la pression réelle est déterminée à partir de l'information de référence.

Avantageusement, ledit capteur comprend un premier élément piézo-électrique réagissant en flexion et/ou en traction/compression aux déplacements d'un premier capteur-plaque en contact direct avec le corps dudit sujet et produisant une première différence de potentiel fonction desdits déplacements du premier capteur-plaque.

Ce capteur présente notamment l'avantage d'un faible encombrement et d'une bonne sensibilité.

Ils sont de plus passifs, et sans nocivité physiologique.

De façon à compenser les mouvements subis par ce premier élément piézo-électrique, le tensiomètre comprend avantageusement un second élément piézo-électrique de compensation réagissant en flexion et/ou en traction/compression aux déplacements d'un second capteur-plaque isolé du corps dudit sujet et produisant une seconde différence de potentiel fonction desdits déplacements du second capteur-plaque.

De façon avantageuse, lesdits premier et second éléments piézo-électriques sont sensiblement identiques et placés en superposition espacée. Ainsi, ils subissent sensiblement les mêmes mouvements.

Dans un mode de réalisation préférentiel de l'invention, ledit premier et/ou ledit second éléments piézo-électriques sont placés au-dessus et/ou au-dessous d'un évidement ménagé dans un circuit imprimé et fixés audit circuit imprimé par leurs extrémités, à l'aide d'une colle conductrice.

Selon un second mode de réalisation avantageux de l'invention, ledit premier et/ou ledit second éléments piézo-électriques est maintenu par l'une de ses deux extrémités, la seconde extrémité étant libre.

La sensibilité de ces éléments est alors améliorée.

Dans ce cas, le tensiomètre peut comprendre deux éléments piézo-électriques sensiblement identiques et placés sensiblement en vis-à-vis. Ledit premier capteur-plaque agit avantageusement sur ledit premier élément piézo-électrique par l'intermédiaire d'un ressort.

Avantageusement, le dispositif comprend des moyens de mesure de la pression d'application dudit capteur sur le corps dudit sujet, délivrant un signal représentatif de ladite pression d'application, et lesdits moyens de traitement corrigent ladite information représentative de la tension artérielle en fonction dudit signal représentatif de la pression d'application. En effet, cette pression peut varier au cours du temps. Elle ne doit toutefois pas induire de modifications sur l'interprétation du signal mesuré.

Cela est notamment utile lorsque le dispositif comprend des moyens de positionnement et/ou de fixation par serrage en contact avec le corps dudit sujet dudit boîtier, tel qu'un bracelet.

Dans ce cas, lesdits moyens de mesure comprennent par exemple un capteur de déplacement réagissant à la tension desdits moyens de positionnement et/ou de serrage.

De façon avantageuse, lesdits moyens de traitement comprennent au moins un des moyens appartenant au groupe comprenant :

- des moyens de numérisation dudit signal représentatif de la pression sanguine instantanée,
- des moyens de suppression de bruit par filtrage,
- des moyens de calcul de la pression artérielle relative dudit sujet, et lesdits moyens de calcul de la pression artérielle relative comprennent au moins un des moyens appartenant au groupe comprenant :
- des moyens de détection des extrema dudit signal représentatif de la pression sanguine instantanée sur une période de temps prédéterminée,
- des moyens de contrôle et de correction mettant en oeuvre un algorithme de vraisemblance,
- des moyens de correction de la pression artérielle relative en fonction de la pression exercée par ledit boîtier sur le corps dudit sujet.

Dans un mode de réalisation avantageux, le tensiomètre de l'invention comprend également des moyens de détermination du rythme cardiaque dudit sujet.

Dans ce cas, préférentiellement, lesdits moyens de calcul du rythme cardiaque comprennent des moyens de détermination de la période du fondamental dudit signal représentatif de la pression sanguine instantanée, tels que des moyens de filtrage multicanal comprenant une pluralité de filtres possédant chacun une bande de fréquence distincte, et des moyens de détection du filtre correspondant à ladite période du fondamental.

Le tensiomètre de l'invention peut également comprendre des moyens de détermination d'une information représentative de la période respiratoire et/ou d'une information représentative de l'amplitude respiratoire dudit sujet.

Dans ce cas, ces moyens comprennent préférentiellement :

- des moyens de détection des maxima et/ou des minima dudit signal représentatif de ladite pression sanguine instantanée ;
- des moyens de détermination et de suppression de la valeur moyenne desdits maxima, respectivement minima ;
- des moyens de calcul de la valeur absolue du signal formé par lesdits maxima, respectivement minima, représentatif de l'amplitude respiratoire ;
- des moyens de calcul de la période du signal formé par lesdits maxima, respectivement minima, représentative de la période respiratoire.

Avantageusement, le dispositif de l'invention comprend des moyens de mémorisation d'une série de valeurs de tension artérielle et/ou d'une série de valeurs de rythme cardiaque et/ou d'une série de valeurs représentatives de l'actimétrie et/ou d'une série de valeurs représentatives de la respiration. Il peut également comprendre des moyens d'analyse d'au moins une desdites séries de valeurs, effectuant au moins un des traitements appartenant au groupe comprenant :

- calcul de moyennes et/ou de statistiques ;
- estimation et/ou prédiction de valeurs futures ;
- génération d'alarme en cas d'évolution alarmante.

Il peut de plus intégrer au moins un des moyens appartenant au groupe comprenant :

- moyens de visualisation ;
- horloge ;
- moyens de réveil ;
- moyens de mémorisation ;
- moyens de communication avec une unité de traitement extérieur ;
- moyens de mesure de la température du corps et/ou du tonus musculaire dudit sujet.

L'invention concerne également un procédé de mise en oeuvre du tensiomètre décrit ci-dessus, comprenant une phase d'initialisation et une phase de fonctionnement en continu,
ladite phase d'initialisation consistant à mémoriser au moins une information de référence représentative du rapport entre au moins une mesure de la tension artérielle absolue effectuée par un tensiomètre de référence, et au moins une mesure dudit signal représentatif de la pression sanguine instantanée effectuée par ledit tensiomètre à mesure en continu, et
ladite phase de fonctionnement en continu consistant à effectuer régulièrement le cycle comprenant les étapes suivantes :

- mesure dudit signal représentatif de la pression sanguine instantanée ;
- détermination de la tension artérielle dudit sujet, en fonction de ladite ou desdites mesures et de ladite ou desdites informations de référence.

Avantageusement, ladite phase d'initialisation comprend les étapes suivantes :

- mesure d'une pression systolique et d'une pression diastolique de référence dudit sujet à l'aide dudit tensiomètre de référence ;
- introduction et mémorisation desdites pressions systolique et diastolique de référence dans ledit tensiomètre à mesure en continu ;
- mesure dudit signal représentatif de la pression sanguine instantanée sur un laps de temps prédéterminé pour au moins deux valeurs prédéterminées de pression dudit tensiomètre sur la peau dudit sujet ;
- détermination de l'amplitude du signal mesuré pour chacune desdites valeurs prédéterminées de pression dudit tensiomètre ;
- calcul d'une fonction mathématique représentative de la variation de l'amplitude du signal mesuré en fonction de la pression dudit tensiomètre sur la peau du sujet.

Le procédé peut de plus comprendre une étape de validation de l'initialisation, consistant à :

- calculer un premier coefficient de forme de référence à partir desdites pressions de référence ;
- déterminer un second coefficient de forme mesuré à partir dudit signal mesuré ;
- comparer lesdits premier et second coefficients de forme.

De façon préférentielle, ladite étape de détermination de la tension artérielle comprend les étapes de :

- détermination de l'amplitude du signal mesuré ;
- mesure de la valeur de la pression dudit tensiomètre sur la peau dudit sujet ;
- calcul de la pression systolique et de la pression diastolique.

Le procédé comprend également avantageusement une étape de validation dudit cycle de fonctionnement en continu, consistant à :

- calculer un premier coefficient de forme de référence à partir desdites pressions de référence ;
- déterminer un second coefficient de forme mesuré à partir dudit signal mesuré ;

- comparer lesdits premier et second coefficients de forme ;
- reprendre la phase d'initialisation si lesdits premier et second coefficients de forme sont différents.

Dans un mode de réalisation particulier, ladite étape de calcul de la pression systolique et de la pression diastolique consiste à calculer les valeurs suivantes :

$$SBP = (3.k-1).Y/q$$

$$DBP = (3.k-2).Y/q$$

ou :    SBP est la pression systolique ;
DBP est la pression diastolique ;
Y est l'amplitude du signal mesuré ;
k est le coefficient de forme mesuré ;
q est un coefficient d'étalonnage dudit tensiomètre.

D'autres caractéristiques et avantages de l'invention apparaîtront à la lecture de la description suivante d'un mode de réalisation préférentiel de l'invention, donnée à titre illustratif et non limitatif, et des dessins annexés, dans lesquels :

- la figure 1 est un schéma synoptique présentant le principe général du dispositif de l'invention ;
- la figure 2 illustre un premier mode de réalisation avantageux de l'invention, dans lequel des capteurs piézo-électriques sont directement collés au circuit imprimé ;
- la figure 3 est le schéma électrique équivalent des capteurs piézo-électriques de la figure 2 ;
- la figure 4 présente les différents capteurs mis en oeuvre selon l'invention, dans le cas d'un dispositif se présentant sous la forme d'une montre-bracelet ;
- la figure 5 est un schéma synoptique détaillé d'un mode de réalisation avantageux de l'invention ;
- la figure 6 présente un exemple d'un signal de pression artérielle ;
- les figures 7 et 8 présentent deux exemples de courbes de l'amplitude du signal mesuré (Y) et du coefficient de forme (k) respectivement, en fonction de la pression (c) effectuée par le boîtier de l'invention ;
- la figure 9 présente une famille de courbes Y = f(c) (figure 7) correspondant à différents états d'un sujet ;
- la figure 10 est un synoptique présentant un mode de réalisation préférentiel du module de calcul de la tension artérielle absolue de la figure 5 ;
- la figure 11 illustre un second mode de montage des cellules piézo-électriques du dispositif de l'invention.

L'invention concerne donc la mesure en continu, et de façon autonome, de la tension artérielle d'un sujet. Pour ce faire, l'invention repose sur une approche nouvelle pour l'homme du métier, basée sur la détection des variations de la pression sanguine du sujet, en permanence.

A partir de ce signal représentatif de la pression sanguine instantanée, on détermine selon l'invention les extrêmas du signal, qui correspondent aux pressions diastolique et systolique du sujet. Ensuite, à partir des données de référence préalablement enregistrées, on détermine ces pressions diastolique et systolique.

Le tensiomètre de l'invention fonctionnant en continu (c'est-à-dire à intervalles réguliers, pouvant variés de quelques secondes à quelques minutes), il doit être portable, autonome, d'un poids limité, et fonctionner sans intervention de l'utilisateur. Dans le mode de réalisation décrit par la suite, le dispositif de l'invention se présente sous la forme d'un montre-bracelet.

D'autres supports sont également envisageables, tels que, par exemple les bagues, colliers, dispositifs adhésifs,... Plus généralement, l'invention peut prendre place dans un boîtier de taille réduite, qui doit être placé en contact avec la peau du sujet.

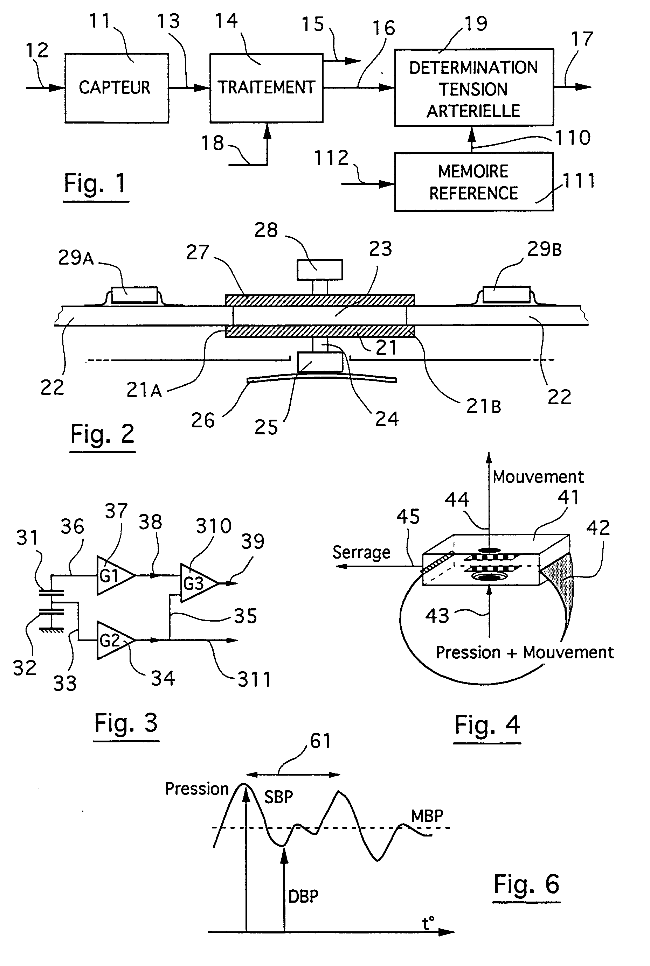

La figure 1 illustre le principe général du dispositif de l'invention.

Un capteur 11, destiné à être appliqué contre le corps du sujet, détecte les pressions 12 de la partie du corps où il est appliqué. Le capteur 11 délivre un signal électrique 13, représentatif de ces pressions.

Le capteur 11 détecte donc d'une part les mouvements du sujet, et d'autre part les pressions de la peau correspondant aux variations de pression sanguine dans un conduit sanguin à proximité du capteur. Ainsi, le signal 13 comporte notamment une information représentative des variations de pression sanguine.

Ce signal 13 est ensuite traité dans des moyens de traitement 14, de façon à en extraire les informations suivantes :

- une information représentative de la tension artérielle 16 ;
- une information représentative du rythme cardiaque 15 (à titre optionnel).

En ce qui concerne l'information 16 de tension artérielle, un problème particulier est rencontré, dès lors que le dispositif de l'invention n'est pas parfaitement immobilisé contre le corps du sujet, par exemple à l'aide d'adhésifs... (c'est notamment le cas si le dispositif est fixé au bras par un bracelet) : la pression appliquée par le boîtier sur la peau du sujet peut varier, soit qu'il se déplace légèrement, soit que le serrage évolue (dans le cas d'un bracelet, celui-ci peut glisser le long du poignet vers un endroit où la circonférence du poignet est plus faible. La pression est alors moins forte).

Cette pression sur la peau a un lien direct avec l'amplitude du signal mesuré. On conçoit en effet que plus le boîtier exerce une pression sur la peau, plus les variations de pression sanguine présentent un niveau élevé. Ces variations de niveau ne doivent bien sûr pas être interprétée comme des variations de la pression sanguine.

Pour éviter ce problème, le dispositif de l'invention propose de tenir compte d'une information 18 représentative de cette pression effectuée par le boîtier. Il peut par exemple s'agir d'une mesure de serrage du bracelet.

En d'autres termes, à pression de serrage du bracelet constante, la variation d'amplitude du signal est directement liée à la pression sanguine. Il suffit de réaliser une mesure d'amplitude du signal issu du capteur pour obtenir l'information sur la pression artérielle en s'assurant de la stabilité de la contrainte induite par le bracelet.

Plus généralement en mesurant la contrainte générée par le bracelet on peut corriger la mesure d'amplitude du signal qui deviendra une grandeur image de la pression sanguine. On utilise donc un capteur mesurant la contrainte 18 induite par le bracelet.

L'information de tension artérielle 16 est une donnée relative (dépendante notamment du sujet porteur du tensiomètre). Pour connaître la tension artérielle réelle, ou absolue, 17, le dispositif de l'invention comprend des moyens 19 de détermination de la tension, qui déterminent les valeurs 17 des pressions systolique et diastolique, à partir de données de référence 110 mémorisées dans des moyens de stockage 111. Ces données de référence 110 ont été obtenues à partir de mesures 112 effectuées (une fois pour toute, au moins tant que l'état du sujet n'évolue pas significativement) à l'aide d'un sphygmomètre classique.

La figure 2 représente (en coupe) un premier exemple de capteur d'activité cardiaque qui peut avantageusement être utilisé dans le dispositif décrit ci-dessus.

Il s'agit d'un capteur piézo-électrique captant le bruit, c'est-à-dire les changements de pression, provoqué par le passage du sang dans les veines, par exemple au niveau du poignet.

Un élément 21 en céramique piézo-électrique est collé par ses deux extrémités $21_A$, $21_B$ à un circuit imprimé 22, dans lequel il a préalablement été ménagé un jour 23 permettant à l'élément 21 de se déformer. Cet élément 21 a par exemple une taille de 12 x 4 x 0,7 mm.

La sensibilité de cette cellule piézo-électrique 21 est comprise entre 1,3 et 13 kPa (entre 1 et 10 mmHg).

Une cale 24 est fixée, par collage, au centre de l'élément 21. Sur la cale 24 prend place une plaque 25 en aluminium, ou en tout autre matériau.

Cette plaque 25, qui peut avoir une surface de 7 x 7 mm, se trouve au contact de la peau 26 du sujet, lorsque le dispositif est en place. La large surface de contact permet d'obtenir un signal de mouvement de forte puissance.

Le mouvement de la peau est transmis à l'élément 21, par l'intermédiaire de la cale 24. Cela induit une flexion de l'élément, faisant apparaître une différence de potentiel entre les deux faces de l'élément 21. C'est cette différence de potentiel qui est fournie au module 14 de traitement du signal de la figure 1.

Avantageusement, le capteur comporte un second groupe élément piézo-électrique 27 et plaque 28, isolé du corps. Ainsi la plaque libre 28 fournit un signal reflétant les mouvements du bras, mouvements qui induisent des signaux non utiles (parasites) dans le premier capteur. Une réduction de ces parasites peut être obtenue en combinant les signaux délivrés par les deux éléments 21 et 27 (soustraction du second au premier).

Le second groupe, ou "capteur de bruit parasite", peut également être positionné de façon différente, au niveau de la fixation du bracelet sur le boîtier. La céramique travaille alors en traction-compression, et non plus en flexion, et capte ainsi mieux les perturbations produites par les variations de pression sur le premier capteur (toujours appliqué sur la peau) tout en étant isolé du signal utile (rythme cardiaque).

Dans le mode de réalisation de la figure 2, les deux bilames (ou éléments piézo-électriques) 21 et 27 sont identiques et compensées en accélération. Elles sont montées en opposition.

De façon à simplifier le montage et à limiter l'encombrement, les éléments 21 et 27 sont collés directement, par leurs extrémités, au circuit imprimé 22, à l'aide d'une colle conductrice. Ainsi, aucun moyen de calage ou de fixation n'est nécessaire, ni aucun moyen de câblage. D'autres techniques, plus classiques, peuvent également être utilisées.

Dans le même objectif de simplification du montage et de limitation de l'encombrement, les différents circuits électriques sont avantageusement des composants $29_A$, $29_B$ montés en surface (C.M.S).

Sous l'effet de l'accélération, on considère que les deux bilames ne se compensent qu'à 20%. On utilisera donc le signal de l'autre cellule pour corriger toute perturbation extérieure, sachant que les réponses impulsionnelles de chaque cellule sont identiques à un coefficient de proportionnalité près.

Le capteur peut donc être représenté, électriquement, par le schéma équivalent de la figure 3, associé à des amplificateurs.

Les cellules 21 et 27 correspondent à deux capacités en série 31 et 32. Le signal 33 prélevé sur la capacité 32 est amplifié par l'amplificateur 34, qui délivre un signal 35 représentatif des mouvements du sujet.

Le signal 36 prélevé sur la capacité 31 est également amplifié par un amplificateur 37 délivrant un signal 38 correspondant aux mouvements et aux variations de la pression artérielle.

Pour obtenir un signal 39 représentatif de la pression artérielle, le signal 35 (mouvements) est soustrait du signal 38 (mouvements + pression artérielle) par un différenciateur 310.

Si le dispositif prend également en compte l'actimétrie du sujet, le signal 35 est transmis (311) aux moyens de traitement. Dans ce cas, le signal 13 de la figure 1 correspond à la combinaison des signaux 39 et 311.

Les gains des amplificateurs sont, à titre indicatif, de l'ordre de :

- amplificateur 37 : G1 = 50
- amplificateur 34 : G2 = 200
- différenciateur 310 : G3 = 1.

Les gains G1 et G2 sont différents car les sensibilités des deux capteurs sont différentes : du fait que la seconde cellule piézo-électrique est isolée, alors que la première subit la pression du poignet. Le gain G3 est choisi de façon à délivrer un signal présentant une bonne dynamique pour la conversion analogique/numérique.

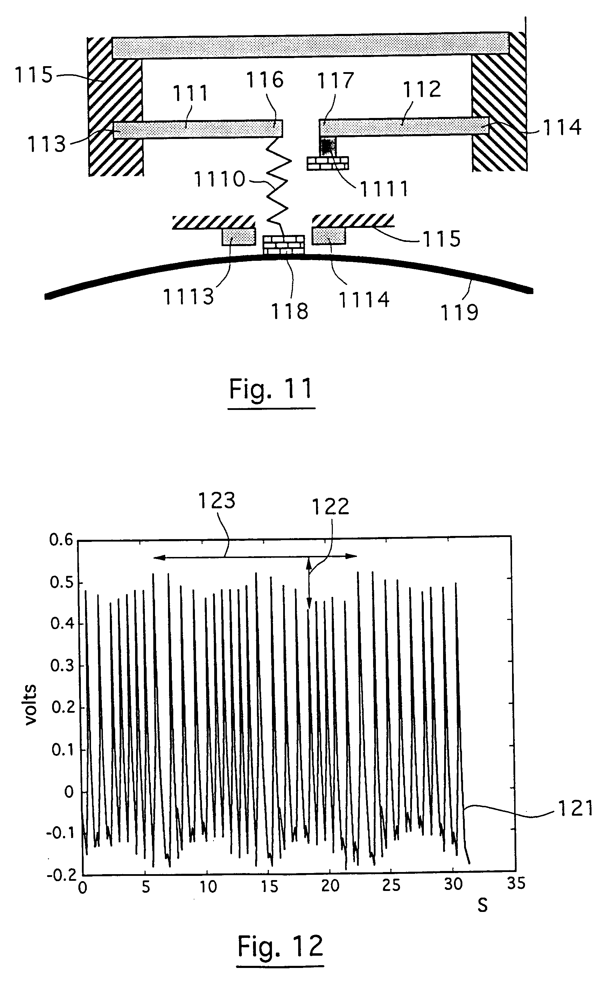

La figure 11 illustre un autre montage avantageux des capteurs du dispositif de l'invention, selon lequel les celules piézo-électriques 111 et 112 ne sont plus superposées, mais placées en vis-à-vis. Elles sont chacune maintenue par l'une de leurs extrémités 113, 114, par exemple par moulage dans le chassis 115 du dispositif. L'autre extrémité 116, 117 de chaque cellule est libre.

Une plaque 118 est placée en contact avec la peau 119 du porteur. Elle est reliée à la cellule 111 par un ressort 1110, lui transmettant ainsi le mouvement de la peau.

Une plaque 1111 est placée à l'extrémité 117 de la seconde cellule 112, qui joue le rôle de capteur de bruit, de façon que les mouvements détectés soient similaires à ceux subis par la première cellule 111.

Des capteurs de pression 1113 et 1114 placés entre la peau 119 et le dessous du boîtier 1115 permettent de mesurer la pression de serrage du bracelet. Ils permettent avantageusement d'assurer une mesure fiable des fluctuations de la pression de serrage jusqu'à 4 kPa (30 mm de Hg). Ce sont par exemple des résistances déposées, telles que les résistances MOTOROLA (marque déposée) référencées MPX2040D.

Les cellules piézo-électriques peuvent être réalisées à partir de la céramique PXE 5 distribuée par RTC (marque déposée).

Les avantages de ce montage par rapport au précédent sont notamment :

- l'amélioration de la sensibilité,
- la similitude du montage des cellules,
- la meilleure fiabilité mécanique,
- meilleure résistance à des pressions extérieures (du fait de la butée 1113 qui limite la course de la pièce 1118).

La figure 4 présente de façon schématique un dispositif selon l'invention, au format d'une montre-bracelet.

Ainsi qu'on l'a déjà mentionné, le dispositif global est avantageusement intégré dans un boîtier 41 maintenu au poignet à l'aide d'un bracelet 42.

Trois informations doivent donc être mesurées :

- l'ensemble pression + mouvement 43 ;
- le mouvement seul 44 ;
- la contrainte 45 effectuée par le bracelet sur le poignet (serrage).

La mesure des données 43 et 44 a été décrite en liaison avec les figures 2 et 3. La cellule de mesure de la contrainte 45 doit être sensible à la composante continue. Plusieurs architectures sont possibles, et par exemple :

- un capteur passe-bas mesurant la composante continue et les fluctuations de la contrainte avec des sensibilités différentes ;
- un capteur passe-bas ne mesurant que la composante continue exercée par le poignet sur le capteur de pression artérielle ;
- un capteur passe-bas mesurant la contrainte du bracelet.

Sa dynamique doit être par exemple de l'ordre de 13 kPa (100 mmHg). Cependant la détection de la contrainte 45 peut se faire à l'aide d'un capteur force ou un capteur pression moins sensible que le capteur de pression artérielle (par exemple une jauge piézo-résistive), ou à l'aide d'un capteur déplacement sachant que celui-ci est fonction de la contrainte appliquée sur le poignet (l'allongement du bracelet correspond en effet à la position du bracelet, donc à la pression effectuée par le poignet sur le capteur).

Dans ce dernier cas, un capteur de déplacement est fixé d'une part au boîtier 41, et d'autre part au bracelet 42. La tension subie par ce capteur est directement représentative du serrage.

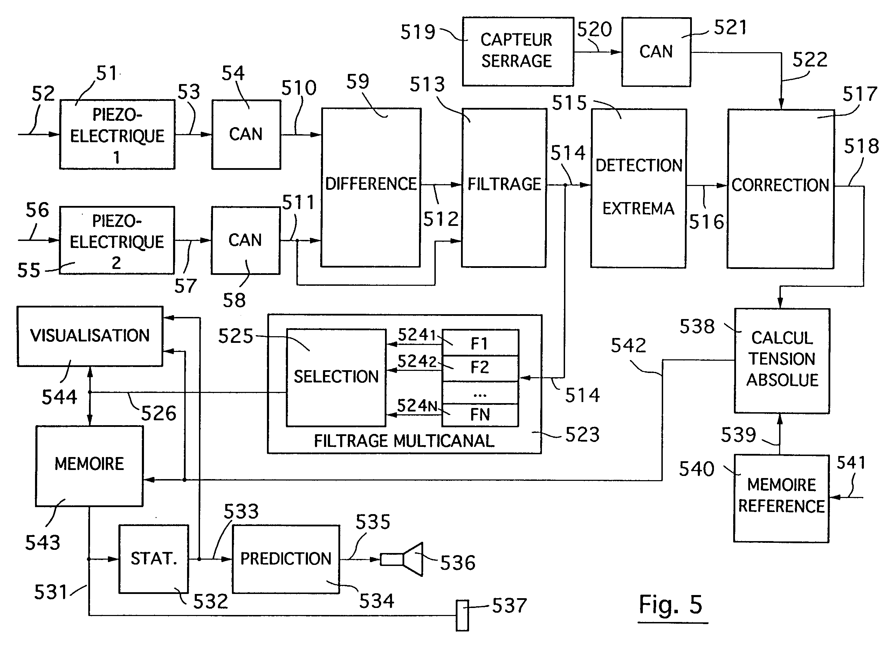

On présente maintenant un mode de réalisation particulier de l'invention, en liaison avec le schéma synoptique de la figure 5.

Un premier élément piézo-électrique 51 détecte donc des déplacements 52 correspondant aux mouvements et à la pression artérielle. Il génère un signal électrique correspondant 53 qui est numérisé, à l'aide d'un convertisseur analogique/numérique (CAN) 54.

De même, un second élément piézo-électrique 55 détecte les mouvements seuls 56. Le signal électrique correspondant 57 est également numérisé, par le convertisseur analogique/numérique 58.

Les deux CAN 54 et 58 échantillonnent les signaux par exemple à un rythme de 64 mesures/s. Ils peuvent avantageusement constituer deux voies d'un convertisseur se chargeant également de la conversion de la contrainte de serrage.

On effectue ensuite la différence 59 entre les signaux numérisés 510 et 511, pour obtenir le signal numérique 512 représentatif de la pression artérielle seule.

Dans la pratique, cette soustraction peut être double :

- on effectue tout d'abord une soustraction câblée, telle qu'illustrée en figure 4. En général, le signal résultant reste perturbé par les mouvements ;
- on réalise donc une seconde soustraction logicielle. Pour ce faire, un troisième convertisseur analogique/numérique numérise le signal "mouvements + pression artérielle" délivré par le premier élément piézo-électrique. Le traitement consiste alors à rechercher les corrélations entre les deux signaux numérisés 511 et 512, puis à supprimer du signal "pression artérielle" ce qui correspond aux mouvements.

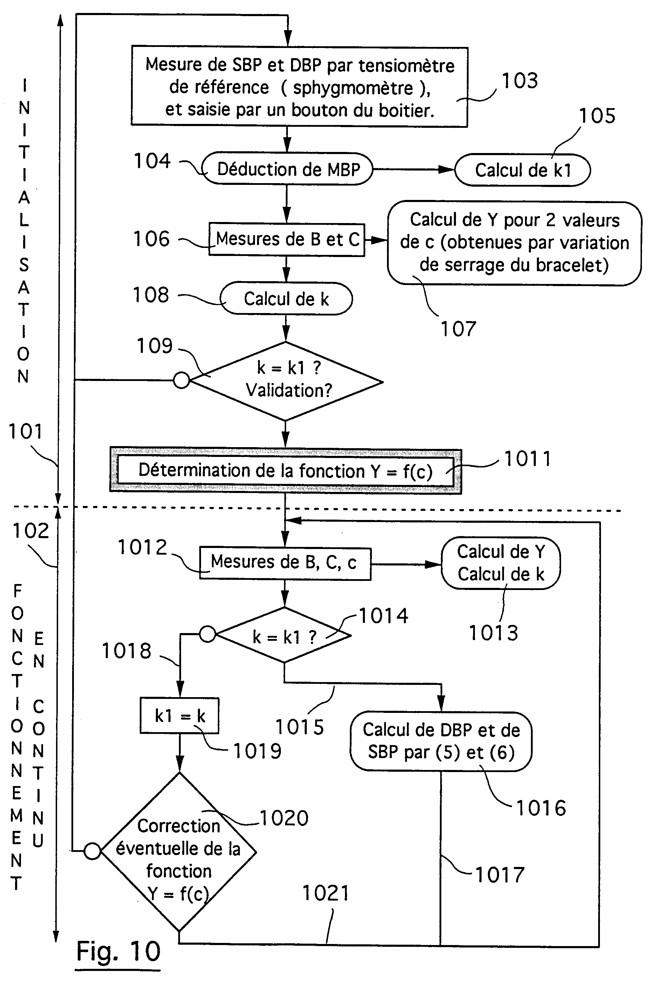

Le signal 512 est donc transmis à un module 513 de linéarisation, de filtrage passe-bas et/ou filtrage adaptatif (recherche et suppression des corrélations entre les signaux 511 et 512) et de mise à l'échelle. La mise à l'échelle consiste à multiplier la valeur reçue par un coefficient de sensibilité représentatif du dispositif. Le signal correspondant 514 peut par exemple être tel que représenté en figure 6.

Sur la figure 6, qui présente l'évolution de la pression artérielle en fonction du temps, on peut distinguer :

- la pression systolique (SBP) ;
- la pression diastolique (DBP) ;
- la pression artérielle moyenne (MBP).

Pour connaître ces différentes pressions on recherche les pics dans le signal 514, à l'aide d'un module 515 de détection des extrêma, sur une période paramétrable de quelques secondes. Connaissant ces extrêma 516, un module de correction 517 détermine une information 518 représentative de la tension artérielle relative du sujet (comprenant au moins une des données : SBP, DBP, MBP). La correction comprend deux niveaux :

- application d'un algorithme de vraisemblance (permettant notamment d'éliminer les données visiblement erronées) ;
- correction fonction de la contrainte exercée par le bracelet (serrage).

Pour ce deuxième niveau de correction, on met en oeuvre un capteur 519 de serrage (ou de pression), délivrant un signal 520 qui est numérisé (521) avant d'être transmis (522) au module 517 de correction.

L'information 518 représentative de la tension artérielle relative est alors transmise à un module 538 de calcul de la tension artérielle absolue. Pour ce faire, il tient compte d'informations de référence 539 stockées dans une mémoire 540. Dans la pratique, le module 540 n'est pas une simple mémoire. Il effectue également un traitement d'initialisation, destiné à calculer les informations 539 à partir de données 541 de tension mesurée de façon externe.

Un exemple de traitement d'initialisation, ainsi que de traitement effectué par le module 538 de calcul de la tension absolue, est présenté par la suite, en liaison avec la figure 10.

Ce module 538 délivre donc finalement l'information 542 de tension artérielle, comprenant les pressions systolique (SBP), diastolique (DBP) et éventuellement moyenne (MBP). Cette information 542 peut être stockée dans une mémoire 543, et également visualisée, à l'aide d'un écran de visualisation 544, par exemple à cristaux liquides.

Par ailleurs, on peut également déterminer (de façon optionnelle) le rythme cardiaque (ou pouls), à partir de l'information 514 de tension artérielle. Cette opération consiste à mesurer la période du fondamental (61, figure 6) contenu dans le signal de pression artérielle.

Plusieurs méthodes sont utilisables et nécessitent des moyens de calcul et de mémoires différents. Avec un traitement fréquentiel, on pourra utiliser une analyse spectrale ou une méthode dérivée. Avec un traitement dans le domaine temporel on utilisera un calcul d'autocorrélation à durée variable par exemple.

En remarquant que la bande d'analyse est étroite et qu'il y a uniquement recherche du fondamental, une approche de calcul par filtrage multicanal 523 est souhaitable. En effet la bande d'analyse est de 30 battements/s à 240 battements/s soit 0,5Hz à 4Hz.

Cette méthode a la particularité d'être peu gourmande en capacité mémoire et en nombre d'opérations à exécuter compte tenu des fréquences de chaque canal $524_1$ à $524_N$ qui peuvent être 0,5 0,66 0,8 1 1,33 2 4 Hz, correspondant à des rythmes cardiaques de 30, 40, 48, 60, 80, 120 et 240 battements/mn.

A chaque canal correspond un débit sous-multiple de la cadence maximale. Ainsi on n'utilise qu'un seul gabarit de filtre. A la sortie de chaque canal $524_1$ à $524_N$ on calcule (525) l'énergie du signal et la période du signal filtré, par comptage. On obtient donc un spectre de raies d'énergie et une fonction de périodes calculées. Une stratégie de sélection 525 détermine le canal le plus fiable, et délivre l'information correspondante 526 qui peut également être mémorisée (543) et/ou visualisée (544).

Avantageusement, à partir du choix du canal, on sélectionne le début de chaque période de pouls. On peut corriger les écarts de valeur des différentes périodes par moyennage. On obtient une courbe moyennée facile à exploiter. On déduit en particulier les valeurs des extrema et la pression artérielle moyenne.

D'autres données physiologiques peuvent encore être déduites du signal de pression sanguine, en particulier en ce qui concerne les paramètres respiratoires.

En effet, ainsi que l'on peut le constater sur la figure 12, la respiration module l'amplitude du signal 121 de pression sanguine.

Il est donc possible de déterminer, à partir d'une extraction des maxima et/ou des minima de ce signal, l'amplitude respiratoire 122 et la période respiratoire 123. Cela permet également, le cas échéant, de détecter les apnées.

Plus précisément, le traitement consiste à chercher les maxima du signal de pression sanguine (amplitude et coordonnées dans le temps). Puis, on supprime la valeur moyenne et, en détectant les passages à zéro du signal, on calcule la période respiratoire et son inverse, la fréquence. Sur la durée de cette période, on calcule la valeur moyenne du signal redressé, qui est une bonne image de l'amplitude respiratoire. Cette analyse est faite par exemple sur une fenêtre d'environ 8 secondes.

On peut opérer de même avec les minima, et moyenner les résultats obtenus entre les minima et les maxima.

Les données stockées 531 permettent d'effectuer des stastistiques 532, dont les résultats 533 peuvent être visualisés, mais surtout utilisés dans un module 534 de prédiction. Il est ainsi possible de générer un signal d'alarme 535, produisant un signal sonore 536, lorsque l'évolution de la pression artérielle et/ou du rythme cardiaque et/ou de la respiration présente des caractéristiques dangereuses pour le sujet. Il est à noter que ce module de prédiction est très important, car ce ne sont pas les valeurs absolues des signaux mais leurs évolutions dans le temps qui sont significatives.

Pour des analyses médicales plus poussées, le dispositif peut comprendre des moyens de connexion 537, qui permettent de transmettre le contenu de la mémoire 530 vers une unité de traitement externe. Eventuellement la liaison peut être hertzienne.

Par exemple, la liaison HF peut mettre en oeuvre une modulation d'amplitude (mode ASK). L'émetteur (dans le boîtier) comprend par exemple un oscillateur local à onde de surface vibrant à 224,5 MHz. A cette fréquence, l'antenne d'émission est de dimensions réduites (quelques cm de côté) et peut être réalisée sur le circuit imprimé.

Après la transmission d'une mesure, l'émetteur est mis en veille, ce qui réduit la consommation de courant. La puissance de l'émetteur peut être inférieure à 5 mW et sa portée de quelques mètres.

Pour le récepteur (dans les moyens externes), on pourra choisir un récepteur de type 'super-réaction' avec un oscillateur local à 213,8 MHz et une amplification intermédiaire à 10,7 MHz suivi d'un décodage.

Avant de présenter un mode de calcul avantageux de la tension artérielle absolue, on décrit maintenant les observations à la base de ce calcul.

Soit r une fonction représentative de la perte de charge vue par le capteur (r est une fonction complexe due à la consistance des muscles, à l'état des conduits sanguins...).

Dans le cadre de l'invention, on considère que r est fonction de la contrainte de fixation mais pas de la variation de la structure entre le capteur et le conduit sanguin, car la contrainte (c) de fixation absolue est grande devant les variations de structure du poignet au cours d'un mouvement. Donc la fonction r peut être connue et est unique. On peut donc déterminer la relation entre la contrainte c de fixation et r pour un patient donné. Ainsi SBP et DBP peuvent être atteintes par la connaissance de la variation de c. Pour obtenir les pressions vraies il suffit alors de référencer le capteur à un instant donné à partir d'un tensiomètre étalon.

On commence par déterminer la relation entre c et l'amplitude Y du signal délivré par le capteur. Y est proportionnel à la différence entre SBP et DBP, soit:

$$Y = q^*(SBP-DBP) \qquad (1)$$

avec q facteur d'étalonnage
ainsi que le coefficient de forme k. k peut être défini par la relation $k = (MBP-DBP)/(SBP- DBP)$ (2) .

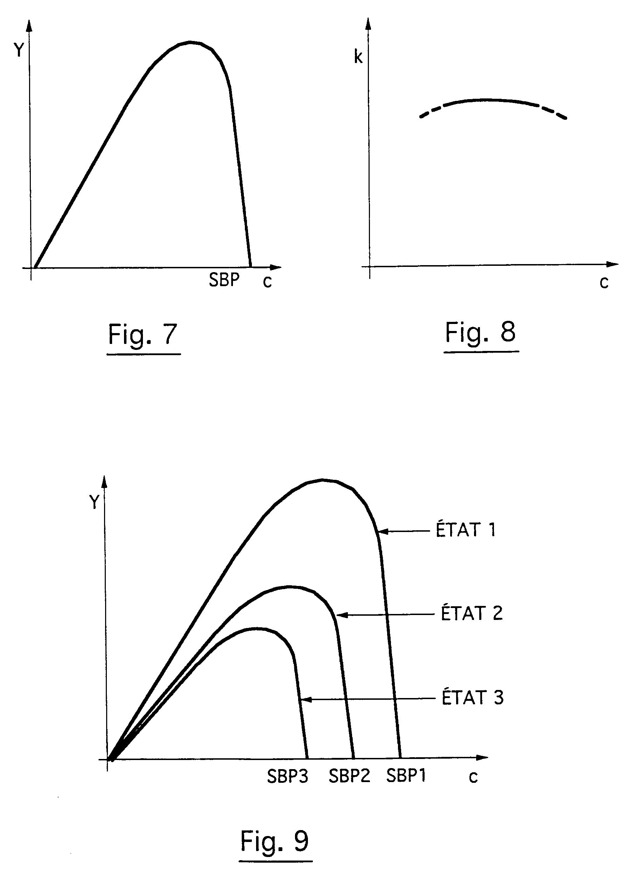

Les figures 7 et 8 présentent des exemples caractéristiques des variations de Y et k respectivement, en fonction de c.

Lorsque c augmente, Y augmente jusqu'à un maximum. La courbe de la figure 7 est représentée jusqu'à SBP, mais cette valeur ne peut être obtenue que par un garrot.

On a donc accès à deux paramètres Y et k. La valeur de k est liée à la structure supportant le capteur et aux caractéristiques des vaisseaux sanguins. L'évolution de k est liée à l'état de santé de la personne.

Dans la mesure où k est constant il est possible de calculer SBP et DBP car Y peut être représenté par une famille de courbes ayant l'aspect illustré en figure 9.

A l'état i d'une personne et pour une contrainte c connue correspondent les pressions SBP, DBP et MBP sont liées par une relation mathématique considérée comme fiable :

$$DBP = (3MBP-SBP)/2 \qquad (3).$$

Cette relation (3) est décrite et justifiée dans le document "Determination of Clinical Accuracy and Nocturnal Blood Pressure Pattern by New Portable Device for Monitoring Indirect Ambulatory Blood Pressure", par Yutaka IMAI, Keishi ABE, Shuichi SASAKI, Naoyoshi MINAMI, Masanori MUNAKATA, Hiroshi SEKINO, Minoru NIIIEI, et Kaoru YOSSHINAGA (American Journal of Hypertension, Inc ; 1990).

A partir des relations (1) et (2) on obtient :

$$DBP = SBP^* (1-(1/(3^*k-1))) \qquad (4)$$

d'où :

$$SBP = (3^* k-1)^*Y/q \qquad (5)$$

$$DBP = (3^* k-2)^*Y/q \qquad (6)$$

La relation (6) montre que la valeur de k est comprise entre 0.66 et 1. En cas de variation de k, l'appareil établit une correction sur Y.

On décrit maintenant, en liaison avec la figure 10, un mode de fonctionnement avantageux de l'invention.

Ainsi qu'on l'a déjà mentionné, le processus de calcul selon l'invention comprend deux phases : une phase d'initialisation 101 et une phase de fonctionnement en continu 102.

La phase d'initialisation 101 à lieu en principe une seule fois, lors de l' acquisition du tensiomètre. Elle consiste à personnaliser ce tensiomètre au sujet, en fonction de ses caractéristiques physiologiques.

Il faut donc noter que le tensiomètre de l'invention est affecté à une personne donnée. Si le porteur change (par exemple dans le cas d'un médecin prescrivant le port de la montre à différents patients successivement), il est nécessaire de reprendre la phase d'initialisation. Il en est de même si les caractéristiques physiologiques du sujet (c'est-à-dire son état de santé) évoluent.

Pour initialiser le tensiomètre, il est tout d'abord nécessaire de réaliser une mesure 103 de la tension artérielle (SBP et DBP) à l'aide d'un appareil externe de référence, tel qu'un sphygmomètre classique. Les données mesurées SBP et DBP sont ensuite introduites dans le tensiomètre de l'invention, par exemple à l'aide d'un ou plusieurs boutons de saisie placés sur le boîtier.

A partir de deux données SBP et DBP, on calcule (104) la pression artérielle moyenne (MBP), selon l'équation :

$$MBP = (2DBP + SBP)/3$$

qui est déduite de l'équation (3).

Connaissant MBP, SBP et DBP, on calcule (105) un coefficient de forme de référence k1, selon l'équation (2).

Dans le même temps (c'est-à-dire dans un intervalle de temps suffisamment réduit pour que la tension artérielle du sujet n'évolue pas), on effectue deux mesures 106 de la pression artérielle instantanée, sur un laps de temps prédéterminé, pour deux contraintes c de serrage de valeurs connues.

A partir de ces mesures, on détermine (107) l'amplitude de Y du signal, en repérant les extrêmes dans le signal mesuré, ainsi que cela a déjà été décrit.

Le minimum de ce signal correspond à DBP, et le maximum à SBP.

Par ailleurs, on détermine également, à partir de ce signal, la pression stabilisée, correspondant à l'instant où l'effet de la surpression du coeur est terminé. Cette valeur correspond à MBP. A partir de ces trois valeurs déterminées sur le signal, on peut calculer (108) un second coefficient de forme k, toujours conformément à l'équation (2).

Si les mesures sont correctes, k doit être égal à k1. On compare donc k et k1 (109). Si le résultat de la comparaison est négatif, l'initialisation est reprise (1010).

Sinon, l'initialisation continue, par la détermination (1011) de la fonction Y = f(c) . Pour ce faire, on connaît trois valeurs de la courbe : les deux points mesurés (107) et le point origine, par lequel passe toutes les courbes Y = f(c) . Le cas échéant, plus de deux mesures peuvent être effectuées. On connaît par ailleurs l'allure générale de la courbe (cf figure 8). La détermination de la fonction peut par exemple correspondre, dans la pratique, à une sélection d'une fonction parmi un ensemble de fonctions pré-enregistrées.

Cette détermination 1011 termine la phase d'initialisation 101.

En fonctionnement en continu 102, on effectue régulièrement une mesure 1012 de la pression artérielle instantanée, à l'aide des capteurs piézo-électriques (mesures 39 et 311, figure 3) et de la contrainte de serrage (mesure c).

Ces mesures permettent de calculer (1013) les valeurs correspondantes de Y et k, de la même façon que présentée précédemment pour les étapes 107 et 108 de la phase d'initialisation 101.

On compare (1014) alors la valeur de k calculée avec la valeur de référence k1. Si le résultat de la comparaison est positif (1015), on calcule (1016) les valeurs de DBP et SBP, à l'aide des équations (5) et (6). Ces valeurs sont affichées et/ou mémorisées. On reboucle ensuite (1017) sur l'étape de mesure 1012. La durée du cycle peut être variable, et on peut également prévoir des moyens d'activation manuelle du cycle, si nécessaire.

Si le résultat de la comparaison est négatif (1018), on modifie (1019) la valeur de référence k1, qui est fixée égale à la valeur du k calculée.

On décide ensuite (1020) d'une éventuelle correction de la fonction Y = f(c) . Si cette correction est possible, on l'effectue, puis on reboucle (1021) sur l'étape 1012 de mesure. Sinon, il est nécessaire de reprendre (102) la phase d'initialisation.

L'invention peut trouver des applications dans de nombreux domaines. Elle peut par exemple être utilisée en tensiomètre-pulsemètre. Dans ce cas le porteur peut suivre son activité cardiaque pour son information personnelle ou les informations peuvent être transmises à une unité de traitement externe, par liaisons téléphoniques ou radioélectriques. Dans ce dernier cas le capteur porté sous forme de montre peut inclure un petit émetteur et agir en alarme par l'intermédiaire d'un relais connecté au réseau téléphonique.

Une application médicale est par exemple le suivi en continu de la tension artérielle (prise de connaissance de la forme de l'onde pression artérielle et de sa valeur).

Le tensiomètre peut également être utilisé pour détecter la somnolence. Ce type d'application peut avoir des débouchés dans de nombreux secteurs (conduite automobile, poste de travail ou de surveillance). L'état de somnolence est un état transitionnel entre l'éveil et le sommeil lent léger ou stade 1. L'activité cardiaque est modifiée et le traitement de nouvelles informations saisies par un capteur facilement portable serait très apprécié. La validation de la détection peut être réalisée par un REM à électrodes détectant le mouvement rapide des paupières qui est un reflet de la somnolence.

L'invention peut également être utilisé dans un dispositif de reconnaissance de phases du sommeil, tel que celui décrit dans la demande conjointe intitulée "Dispositif de reconnaissance de phases du sommeil, et application correspondante", portant le numéro FR-2 700 689.

## Revendications

1. Tensiomètre à mesure en continu de la tension artérielle d'un sujet, intégrant dans un boîtier (41) portatif autonome un capteur (11) réagissant aux variations (12) de la pression sanguine dudit sujet, délivrant un signal (13) représentatif de ladite pression sanguine instantanée,
   caractérisé par

   - des moyens (111) de mémorisation permanente d'au moins une information de référence (110) fonction de la tension artérielle (112) dudit sujet dans un état prédéterminé, ladite ou lesdites informations de référence étant relevées à l'aide d'un dispositif de référence, puis inscrites dans lesdits moyens (111) de mémorisation, lors d'une phase d'initialisation dudit tensiomètre,
   - des moyens (14) de traitement dudit signal (13) représentatif de la pression sanguine instantanée, délivrant au moins une information relative (16) représentative des variations de tension artérielle mesurées dudit sujet, et
   - des moyens (19) de détermination d'une valeur absolue de ladite tension artérielle (17) dudit sujet, en fonction de ladite ou desdites informations de référence (110) et de ladite ou desdites informations relatives (16) représentative des variations de tension artérielle mesurées.

2. Tensiomètre selon la revendication 1, caractérisé en ce que ledit capteur (11) comprend un premier élément piézo-électrique (21 ; 111) réagissant en flexion et/ou en traction/compression aux déplacements d'un premier capteur-plaque (25 ; 118) en contact direct avec le corps dudit sujet et produisant une première différence de potentiel fonction desdits déplacements du premier capteur-plaque (25 ; 118).

3. Tensiomètre selon la revendication 2, caractérisé en ce que ledit capteur (11) comprend un second élément piézo-électrique (27 ; 112) de compensation réagissant en flexion et/ou en traction/compression aux déplacements d'un second capteur-plaque (28 ; 1111) isolé du corps dudit sujet et produisant une seconde différence de potentiel fonction desdits déplacements du second capteur-plaque (28 ; 1111).

4. Tensiomètre selon la revendication 3, caractérisé en ce que lesdits premier et second éléments piézo-électriques (21, 27) sont sensiblement identiques et placés en superposition espacée.

5. Tensiomètre selon l'une quelconque des revendications 2 à 4, caractérisé en ce que ledit premier et/ou ledit second éléments piézo-électriques (21, 27) sont placés au-dessus et/ou au-dessous d'un évidement (23) ménagé dans un circuit imprimé (22) et fixés audit circuit imprimé (22) par leurs extrémités (21$_A$, 21$_B$).

6. Tensiomètre selon la revendication 5, caractérisé en ce que ledit premier et/ou ledit second éléments piézo-électriques (21, 27) sont collés audit circuit imprimé (22) par leurs extrémités, à l'aide d'une colle conductrice.

7. Tensiomètre selon l'une quelconque des revendications 2 et 3, caractérisé en ce que ledit premier et/ou ledit second éléments piézo-électriques (111, 112) est maintenu par l'une de ses deux extrémités (113, 112), la seconde desdites extrémités (116, 117) étant libre.

8. Tensiomètre selon la revendication 7, caractérisé en ce qu'il comprend deux éléments piézo-électriques (111, 112) sensiblement identiques et placés sensiblement en vis-à-vis.

9. Tensiomètre selon l'une quelconque des revendications 7 et 8, caractérisé en ce que ledit premier capteur-plaque (118) agit sur ledit premier élément piézo-électrique (111) par l'intermédiaire d'un ressort (1110).

10. Tensiomètre selon l'une quelconque des revendications 1 à 9, caractérisé en ce qu'il comprend des moyens (519) de mesure de la pression d'application dudit capteur (11) sur le corps dudit sujet, délivrant un signal (520) représentatif de ladite pression d'application,
   et en ce que lesdits moyens (14) de traitement corrigent ladite information (16) représentative des variation de tension artérielle en fonction dudit signal (18) représentatif de la pression d'application.

11. Tensiomètre selon l'une quelconque des revendications 1 à 10, caractérisé en ce qu'il comprend des moyens (42) de positionnement et/ou de fixation par serrage en contact avec le corps dudit sujet dudit boîtier (41).

12. Tensiomètre selon la revendication 11, caractérisé en ce que lesdits moyens de positionnement et/ou de serrage comprennent un bracelet (42).

13. Tensiomètre selon la revendication 12 et l'une quelconque des revendications 10 et 11, caractérisé en ce que lesdits moyens de mesure comprennent un capteur (519) de déplacement réagissant à la tension desdits moyens (42) de positionnement et/ou de serrage.

14. Tensiomètre selon l'une quelconque des revendications 1 à 13, caractérisé en ce que lesdits moyens de traitement comprennent au moins un des moyens appartenant au groupe comprenant :

   - des moyens (54) de numérisation dudit signal représentatif de la pression sanguine instantanée,
   - des moyens (513) de suppression de bruit par filtrage,
   - des moyens (515, 517) de calcul de la pression artérielle relative dudit sujet.

15. Tensiomètre selon la revendication 14, caractérisé en ce que lesdits moyens de calcul de la pression artérielle relative comprennent au moins un des moyens appartenant au groupe comprenant :

   - des moyens (515) de détection des extrema dudit signal représentatif de la pression sanguine instantanée sur une période de temps prédéterminée,
   - des moyens (517) de contrôle et de correction mettant en oeuvre un algorithme de vraisemblance,
   - des moyens (517) de correction de la pression artérielle relative en fonction de la pression exercée par ledit boitier sur le corps dudit sujet.

16. Tensiomètre selon l'une quelconque des revendications 1 à 15, caractérisé en ce qu'il comprend également des moyens (523) de détermination du rythme cardiaque dudit sujet.

17. Tensiomètre selon la revendication 16, caractérisé en ce que lesdits moyens de calcul du rythme cardiaque comprennent des moyens (523) de détermination de la période du fondamental dudit signal représentatif de la pression sanguine instantanée.

18. Tensiomètre selon la revendication 17, caractérisé en ce que lesdits moyens de détermination comprennent des moyens (523) de filtrage multicanal comprenant une pluralité de filtres $(524_1$ à $524_N)$ possédant chacun une bande de fréquence distincte, et des moyens (525) de détection du filtre correspondant à ladite période du fondamental.

19. Tensiomètre selon l'une quelconque des revendications 1 à 15, caractérisé en ce qu'il comprend également des moyens (523) de détermination d'une information représentative de la période respiratoire et/ou d'une information représentative de l'amplitude respiratoire dudit sujet.

20. Tensiomètre selon la revendication 19, caractérisé en ce qu'il comprend au moins un des moyens appartenant au groupe comprenant :

   - des moyens de détection des maxima et/ou des minima dudit signal représentatif de ladite pression sanguine instantanée ;
   - des moyens de détermination et de suppression de la valeur moyenne desdits maxima, respectivement minima ;
   - des moyens de calcul de la valeur absolue du signal formé par lesdits maxima, respectivement minima, représentatif de l'amplitude respiratoire ;
   - des moyens de calcul de la période du signal formé par lesdits maxima, respectivement minima, représentative de la période respiratoire.

21. Tensiomètre selon l'une quelconque des revendications 1 à 20, caractérisé en ce qu'il comprend des moyens (543) de mémorisation d'une série de valeurs de tension artérielle et/ou d'une série de valeurs de rythme cardiaque et/ou d'une série de valeurs de période respiratoire et/ou d'une série de valeurs d'amplitude respiratoire.

22. Tensiomètre selon la revendication 21, caractérisé en ce qu'il comprend des moyens (532, 534) d'analyse d'au moins une desdites séries de valeurs, effectuant au moins un des traitements appartenant au groupe comprenant :

   - calcul (532) de moyennes et/ou de statistiques ;
   - estimation et/ou prédiction (534) de valeurs futures ;
   - génération d'alarme (536) en cas d'évolution alarmante.

23. Procédé de détermination de la tension artérielle d'un sujet à l'aide d'un tensiomètre à mesure en continu selon l'une quelconque des revendications 1 à 22, caractérisé en ce qu'il comprend une phase d'initialisation (101) et une phase de fonctionnement en continu (102), ladite phase d'initialisation (101) consistant à mémoriser au moins une information de référence représentative du rapport entre au moins une mesure de la tension artérielle absolue effectuée par un tensiomètre de référence, et moins une mesure dudit signal représentatif de la pression sanguine instantanée effectuée par ledit tensiomètre à mesure en continu, et

ladite phase de fonctionnement en continu (102) consistant à effectuer régulièrement le cycle comprenant les étapes suivantes :

- mesure (1012) dudit signal représentatif de la pression sanguine instantanée ;
- détermination (1016) de la tension artérielle dudit sujet, en fonction de ladite ou desdites mesures et de ladite ou desdites informations de référence.

24. Procédé selon la revendication 23, caractérisé en ce que ladite phase d'initialisation (101) comprend les étapes suivantes :

- mesure (103) d'une pression systolique et d'une pression diastolique de référence dudit sujet à l'aide dudit tensiomètre de référence ;
- introduction et mémorisation (103) desdites pressions systolique et diastolique de référence dans ledit tensiomètre à mesure en continu;
- mesure (106) dudit signal représentatif de la pression sanguine instantanée sur un laps de temps prédéterminé pour au moins deux valeurs prédéterminées de pression dudit tensiomètre sur la peau dudit sujet ;
- détermination (107) de l'amplitude du signal mesuré pour chacune desdites valeurs prédéterminées de pression dudit tensiomètre ;
- calcul (1011) d'une fonction mathématique représentative de la variation de l'amplitude du signal mesuré en fonction de la pression dudit tensiomètre sur la peau du sujet.

25. Procédé selon la revendication 23 et 24, caractérisé en ce qu'il comprend une étape de validation de l'initialisation, consistant à :

- calculer (105) un premier coefficient de forme de référence à partir desdites pressions de référence ;
- déterminer (108) un second coefficient de forme mesuré à partir dudit signal mesuré ;
- comparer (109) lesdits premier et second coefficients de forme.

26. Procédé selon l'une quelconque des revendications 23 à 25, caractérisé en ce que ladite étape de détermination de la tension artérielle comprend les étapes de :

- détermination (1013) de l'amplitude du signal mesuré ;
- mesure (1012) de la valeur de la pression dudit tensiomètre sur la peau dudit sujet ;
- calcul (1016) de la pression systolique et de la pression diastolique.

27. Procédé selon la revendication 26, caractérisé en ce qu'il comprend une étape de validation dudit cycle de fonctionnement en continu, consistant à :

- calculer (105) un premier coefficient de forme de référence à partir desdites pressions de référence ;
- déterminer (1013) un second coefficient de forme mesuré à partir dudit signal mesuré ;
- comparer (1014) lesdits premier et second coefficients de forme ;
- reprendre la phase d'initialisation et/ou le calcul de ladite fonction si lesdits premier et second coefficients de forme sont différents.

28. Procédé selon l'une quelconque des revendications 26 et 27, caractérisé en ce que ladite étape (1016) de calcul de la pression systolique et de la pression diastolique consiste à calculer les valeurs suivantes :

$$SBP = (3.k\text{-}1).Y/q$$

$$DBP = (3.k\text{-}2).Y/q$$

où : SBP est la pression systolique ;
DBP est la pression diastolique ;
Y est l'amplitude du signal mesuré ;
k est le coefficient de forme mesuré ;
q est un coefficient d'étalonnage dudit tensiomètre.

## Claims

1. Sphygmomanometer for continuously measuring the blood pressure of a subject, combining in a self-contained portable case (41) a sensor (11) reacting to variations (12) in the blood pressure of the said subject, delivering a signal (13) representing the said instantaneous blood pressure, characterized in that there are :

- means (111) for continually memorizing at least one reference item of data (110) which is a function of the blood pressure (112) of the said subject in a predetermined state, the said reference item or items of data being recorded with the aid of a reference device and then entered into the said means (111) of memorizing, during an initialization phase of the said sphygmomanometer,

- means (14) for processing the said signal (13) representing the instantaneous blood pressure, delivering at least one relative item of data (16) representing the measured variations in the blood pressure of the said subject, and

- means (19) for determining an absolute value for the said blood pressure (17) of the said subject, as a function of the said reference item or items of data (110) and the said relative item or items of data (16) representing variations in the blood pressure measured.

2. Sphygmomanometer according to claim 1, characterized in that the said sensor (11) comprises a first piezo-electric element (21; 111) reacting in deflection and/or tension/compression to displacements of a first plate-sensor (25; 118) in direct contact with the body of the said subject and producing a first potential difference which is a function of the said displacements of the first plate-sensor (25; 118).

3. Sphygmomanometer according to claim 2, characterized in that the said sensor (11) comprises a second compensating piezo-electric element (27; 112) reacting in deflection and/or tension/compression to displacements of a second plate-sensor (28; 1111) insulated from the body of the said subject and producing a second difference in potential which is a function of the said displacements of the second plate-sensor (28; 1111).

4. Sphygmomanometer according to claim 3, characterized in that the said first and second piezo-electric elements (21,27) are substantially identical and placed so as to be spaced and superimposed.

5. Sphygmomanometer according to one of claims 2 to 4, characterized in that the said first and/or the said second piezo-electric elements (21,27) is/are placed above and/or below a recess (23) provided in a printed circuit (22) and attached to the said printed circuit (22) by their ends (21$_A$.21$_B$).

6. Sphygmomanometer according to claim 5, characterized in that the said first and/or the said second piezo-electric elements (21,27) is/are glued to the said printed circuit (22) by their ends with the aid of a conducting adhesive.

7. Sphygmomanometer according to either of claims 2 or 3, characterized in that the said first and/or the said second piezo-electric elements (111,112) is/are held by one of its/their two ends (113,112), the second of the said ends (116,117) being free.

8. Sphygmomanometer according to claim 7, characterized in that it comprises two piezo-electric elements (111,112) which are substantially identical and placed substantially facing each other.

9. Sphygmomanometer according to either of claims 7 or 8, characterized in that the said first plate-sensor (118) acts on the said first piezo-electric element (111) via a spring (1110).

10. Sphygmomanometer according to any of claims 1 to 9, characterized in that it comprises means (519) for measuring the application pressure of the said sensor (11) on the body of the said subject, delivering a signal (520) representing the said application pressure, and in that the said processing means (14) correct the said data (16) representing variations in blood pressure as a function of the said signal (18) representing the application pressure.

11. Sphygmomanometer according to any of claims 1 to 10, characterized in that it comprises means (42) for positioning and/or attaching by clamping the said case (41) in contact with the body of the said subject.

12. Sphygmomanometer according to claim 11, characterized in that the said means for positioning and/or clamping comprise a bracelet (42).

13. Sphygmomanometer according to claim 12 and either of claims 10 or 11, characterized in that the said means of measurement comprise a displacement transducer (519) reacting to the tension of the said means (42) for positioning and/or clamping.

14. Sphygmomanometer according to any of claims 1 to 13, characterized in that the said processing means comprise at least one of the means belonging to the group comprising :

- means (54) for digitizing the said signal representing the instantaneous blood pressure,

- means (513) for suppressing noise by filtering,

- means (515,517) for calculating the relative blood pressure of the said subject.

15. Sphygmomanometer according to claim 14, characterized in that the said means for calculating the relative blood pressure comprise at least one of the means belonging to the group comprising :

- means (515) for detecting the extrema of the said signal representing the instantaneous blood pressure over a predetermined period of time,

- means (517) for control and correction using a likelihood algorithm,

- means (517) for correcting the relative blood pressure as a function of the pressure exerted by the said case on the body of the said subject.

16. Sphygmomanometer according to any of claims 1 to 15, characterized in that it also comprises means (523) for determining the heartbeat rate of the said subject.

17. Sphygmomanometer according to claim 16, characterized in that the said means for calculating the heartbeat rate comprises means (523) for determining the fundamental period of the said signal representing the instantaneous blood pressure.

18. Sphygmomanometer according to claim 17, characterized in that the said means of determination comprise multi-channel means of filtering (523) comprising a plurality of filters ($524_1$ to $524_N$) each possessing a distinct frequency band, and means (525) for detecting the filter corresponding to the said fundamental period.

19. Sphygmomanometer according to any of claims 1 to 15, characterized in that it also comprises means (523) for determining data representing the respiratory period and/or data representing the respiratory amplitude of the said subject.

20. Sphygmomanometer according to claim 19, characterized in that it comprises at least one of the means belonging to the group comprising :

- means for detecting the maxima and/or minima of the said signal representing the said instantaneous blood pressure;

- means for determining and suppressing the mean value of the said maxima and minima respectively;

- means for calculating the absolute value of the signal formed by the said maxima and minima respectively, representing the respiratory amplitude;

- means for calculating the period of the signal formed by the said maxima and minima respectively, representing the respiratory period.

21. Sphygmomanometer according to any of claims 1 to 20, characterized in that it comprises means (543) for memorizing a series of values of the blood pressure and/or a series of values for the heartbeat rate and/or a series of values for the respiratory period and/or a series of values for the respiratory amplitude.

22. Sphygmomanometer according to claim 21, characterized in that it comprises means (532, 534) for analysing at least one of the said series of values, performing at least one of the processes belonging to the group comprising :

- calculating (532) means and/or statistics;

- estimating and/or predicting (534) future values;

- producing an alarm (536) in the case of an alarming change.

23. Method for determining the blood pressure of a subject with the aid of a continuous measurement sphygmomanometer according to any of claims 1 to 22, characterized in that it comprises an initialization phase (101) and a phase of continuous operation (102),
the said initialization phase (101) consisting of memorizing at least one reference item of data representing the ratio between at least one measurement of the absolute blood pressure taken by a reference sphygmomanometer, and at least one measurement of the said signal representing the instantaneous blood pressure taken by the said continuous measurement sphygmomanometer, and
the said phase of continuous operation (102) consisting of regularly carrying out a cycle comprising the following steps :

- measuring (1012) the said signal representing the instantaneous blood pressure;

- determining (1016) the blood pressure of the said subject as a function of the said measurement or measurements of the said reference item or items of data.

24. Method according to claim 23, characterized in that the said initialization phase (101) comprises the following steps :

- Measuring (103) a reference systolic pressure and a reference diastolic pressure of the said subject with the aid of the said reference sphygmomanometer;

- Entering and memorizing (103) the said reference systolic and diastolic pressures in the said continuous measurement sphygmomanometer;

- Measuring (106) the said signal representing the instantaneous blood pressure over a predetermined period of time for at least two predetermined values of the pressure of the said sphygmomanometer on the skin of the said subject;

- Determining (107) the amplitude of the signal measured for each of the said predetermined pressure values of the said sphygmomanometer;

- Calculating (1011) a mathematical function representing the variation of amplitude of the signal measured as a function of the pressure of the said sphygmomanometer on the skin of the subject.

25. Method according to claim 23 and 24, characterized in that it comprises a validation step of the initialization consisting of :

- calculating (105) a first reference form factor from the said reference pressures;

- determining (108) a second form factor measured from the said measured signal;

- comparing (109) the said first and second form factors.

26. Method according to any of claims 23 to 25, characterized in that the said step of determining the blood pressure comprises the steps of :

- determining (1013) the amplitude of the signal measured;

- measuring (1012) the value of the pressure of the said sphygmomanometer on the skin of the said subject;

- calculating (1016) the systolic pressure and diastolic pressure.

27. Method according to claim 26, characterized in that it comprises a validation step for the said continuously operating cycle, consisting of :

- calculating (105) a first reference form factor from the said reference pressures;

- determining (1013) a second form factor measured from the said measured signal;

- comparing (1014) the said first and second form factors;

- repeating the initialization phase and/or the calculation of the said function if the said first and second form factors are different.

28. Method according to either of claims 26 or 27, characterized in that the said step (1016) of calculating the systolic pressure and the diastolic pressure consists of calculating the following values :

$$SBP = (3.k-1).Y/q$$

$$DBP = (3.k-2).Y/q$$

where :   SBP is the systolic pressure;
DBP is the diastolic pressure;
Y is the amplitude of the signal measured;
k is the form factor measured
q is a calibration factor of the said sphygmomanometer.

**Patentansprüche**

1. Kontinuierlich arbeitendes Blutdruckmeßgerät, das in einem unabhängigen, tragbaren Gehäuse (41) einen Meßfühler (11) besitzt, der auf Blutdruckänderungen (12) der untersuchten Person reagiert, wobei ein für den augenblicklichen Blutdruck repräsentatives Signal (13) emittiert wird, gekennzeichnet durch

- Mittel (111) zum dauerhaften Speichern von mindestens einer Referenzinformation (110) als Funktion des Blutdrucks (112) der untersuchten Person in einem vorgegebenen Zustand, wobei der Referenzwert oder die Referenzwerte mit Hilfe eines Referenzsystems aufgezeichnet und bei einer Initialisierungsphase des Blutdruckmeßgerätes in die Speichermittel (111) geschrieben wird bzw. werden,

- Bearbeitungsmittel (14) des für den augenblicklichen Blutdruck repräsentativen Signals (13), die mindestens eine relative Information (16) zur Verfügung stellen, welche repräsentativ für die gemessenen Blutdruckänderungen der untersuchten Person sind, und

- Mittel (19) zum Ermitteln eines Absolutwertes des Blutdruckes (17) der untersuchten Person, als Funktion des Referenzwertes bzw. der Referenzwerte (110) und der relativen Information(en) (16), die für die Veränderungen des gemessenen Blutdruckes repräsentativ sind.

2. Blutdruckmeßgerät gemäß Anspruch 1, dadurch gekennzeichnet, daß der Meßfühler (11) ein erstes piezoelektrisches Element (21; 111) umfaßt, das durch Biegung und/oder durch Zug/Kompression auf die Bewegungen eines ersten Plattenmeßfühlers (25, 118) reagiert, der den Körper der untersuchten Person berührt, wobei das piezoelektrische Element eine erste Spannung in Abhängigkeit von den Bewegungen des ersten Plattenmeßfühlers (25, 118) verursacht.

3. Blutdruckmeßgerät gemäß Anspruch 2, dadurch gekennzeichnet, daß der Meßfühler (11) ein zweites piezoelektrisches Kompensationselement (27, 112) umfaßt, das durch Biegung und/oder durch Zug/Kompression auf die Bewegungen einer zweiten Meßfühlerplatte (28, 1111) reagiert, die vom Körper der untersuchten Person isoliert ist, wobei das piezoelektrische Element eine zweite Spannung, abhängig von den Bewegungen der zweiten Meßfühlerplatte (28, 1111) verursacht.

4. Blutdruckmeßgerät gemäß Anspruch 3, dadurch gekennzeichnet, daß das erste und das zweite piezoelektrische Element (21, 27) in etwa identisch sind und mit einem Zwischenraum übereinander angebracht sind.

5. Blutdruckmeßgerät gemäß einem der Ansprüche 2 bis 4, dadurch gekennzeichnet, daß das erste und/oder das zweite piezoelektrische Element (21, 27) oberhalb und/oder unterhalb einer in einer gedruckten Schaltung (22) praktizierten Aussparung (23) angebracht sind (ist), und über ihre (seine) Enden (21$_A$, 21$_B$) an der gedruckten Schaltung befestigt sind (ist).

6. Blutdruckmeßgerät gemäß Anspruch 5, dadurch gekennzeichnet, daß die Enden des ersten und/oder des zweiten piezoelektrischen Elementes (21, 27) auf die gedruckte Schaltung (22) mit einem leitenden Kleber aufgeklebt sind.

7. Blutdruckmeßgerät gemäß einem der Ansprüche 2 oder 3, dadurch gekennzeichnet, daß das erste und/oder das zweite piezoelektrische Element (111, 112) an einem Ende (113, 112) befestigt sind (ist), wobei das jeweils zweite Ende (116, 117) frei bleibt.

8. Blutdruckmeßgerät gemäß Anspruch 7, dadurch gekennzeichnet, daß es zwei in etwa identische piezoelektrische Elemente (111, 112) umfaßt, die in etwa einander gegenüber angebracht sind.

9. Blutdruckmeßgerät gemäß einem der Ansprüche 7 oder 8, dadurch gekennzeichnet, daß der erste Plattenmeßfühler (118) über eine Feder (1110) auf das erste piezoelektrische Element (111) wirkt.

10. Blutdruckmeßgerät gemäß einem der Ansprüche 1 bis 9, dadurch gekennzeichnet, daß es über Mittel (519) zum Messen des Anwendungsdruckes des Meßfühlers (11) auf dem Körper der untersuchten Person verfügt, welche ein für diesen Anwendungsdruck repräsentatives Signal (520) senden, und daß die Bearbeitungsmittel (14) die Information (16) korrigieren, welche repräsentativ für die Blutdruckänderungen als Funktion des für den Anwendungsdruck repräsentativen Signals (18) ist.

11. Blutdruckmeßgerät gemäß einem der Ansprüche 1 bis 10, dadurch gekennzeichnet, daß es über Mittel (42) verfügt, mit dem das Gehäuse (41) in Berührung mit dem Körper der untersuchten Person durch Festklemmen positioniert und/oder befestigt werden kann.

12. Blutdruckmeßgerät gemäß Anspruch 11, dadurch gekennzeichnet, daß die Mittel zum Positionieren und/oder zum Festklemmen ein Armband (42) umfassen.

13. Blutdruckmeßgerät gemäß Anspruch 12 und einem der Ansprüche 10 oder 11, dadurch gekennzeichnet, daß die Meßmittel einen Bewegungsmeßfühler (519) umfassen, der auf die Spannung der Positionierungsmittel (42) und/oder der Mittel zum Festklemmen reagiert.

14. Blutdruckmeßgerät gemäß einem der Ansprüche 1 bis 13, dadurch gekennzeichnet, daß die Bearbeitungsmittel mindestens eines der Mittel aus der Gruppe enthalten, die folgendes umfaßt:

- Mittel (54) zum Digitalisieren des für den augenblicklichen Blutdrucks repräsentativen Signals,

- Mittel (513) zur Rauschunterdrückung durch Filterung,

- Mittel (515, 517) zum Berechnen des relativen Arterienblutdrucks der untersuchten Person.

15. Blutdruckmeßgerät gemäß Anspruch 14, dadurch gekennzeichnet, daß die Mittel zum Berechnen des relativen Arterienblutdrucks mindestens eines der Mittel aus der Gruppe enthalten, die folgendes umfaßt:

- Mittel (515) zum Erfassen der Maxima und Minima des für den augenblicklichen Blutdrucks repräsentativen Signals über einen vorgegebenen Zeitraum,

- Kontroll- und Korrekturmittel (517), die einen Wahrscheinlichkeitsalgorithmus anwenden,

- Korrekturmittel (517) für den relativen Arterienblutdruck als Funktion des auf den Körper der untersuchten Person vom Gehäuse ausgeübten Druckes.

16. Blutdruckmeßgerät gemäß einem der Ansprüche 1 bis 15, dadurch gekennzeichnet, daß es ebenfalls Mittel (523) zum Ermitteln des Herzrhythmus der untersuchten Person umfaßt.

17. Blutdruckmeßgerät gemäß Anspruch 16, dadurch gekennzeichnet, daß die Mittel zum Berechnen des Herzrhythmus Mittel (523) zum Ermitteln der Grundschwingungsperiode des für den augenblicklichen Blutdrucks repräsentativen Signals umfassen.

18. Blutdruckmeßgerät gemäß Anspruch 17, dadurch gekennzeichnet, daß diese Feststellungsmittel über Mehrkanal-Filterungsmittel (523) verfügen, welche eine Mehrzahl von Filtern ($524_1$ bis $524_N$) umfassen, die jeweils ein anderes Frequenzband haben, sowie über Mittel (525) zum Erfassen des der Grundschwingungsperiode entsprechenden Filters.

19. Blutdruckmeßgerät gemäß einem der Ansprüche 1 bis 15, dadurch gekennzeichnet, daß es ebenfalls über Mittel (523) zum Feststellen einer für die Atemperiode und/oder einer für die Atmungsamplitude der untersuchten Person repräsentativen Information verfügt.

20. Blutdruckmeßgerät gemäß Anspruch 19, dadurch gekennzeichnet, daß es mindestens eines der Mittel aus der Gruppe enthält, die folgendes umfaßt:

- Mittel zum Erfassen der Maxima und/oder der Minima des für den augenblicklichen Blutdruck repräsentativen Signals;

- Mittel zum Feststellen und zum Unterdrücken des Mittelwertes dieser Maxima bzw. Minima;

- Mittel zum Berechnen des Absolutwertes des aus diesen Maxima bzw. aus diesen Minima gebildeten und für die Atmungsamplitude repräsentativen Signals;

- Mittel zum Berechnen der Periode des von diesen Maxima bzw. Minima gebildeten und für die Atmungsperiode repräsentativen Signals.

21. Blutdruckmeßgerät gemäß einem der Ansprüche 1 bis 20, dadurch gekennzeichnet, daß es über Mittel (543) zum Speichern einer Reihe von Arterienblutdruckwerten und/oder einer Reihe von Herzrhythmuswerten und/oder einer Reihe von Atmungsperiodenwerten und/oder einer Reihe von Atmungsamplitudenwerten verfügt.

22. Blutdruckmeßgerät gemäß Anspruch 21, dadurch gekennzeichnet, daß es über Mittel (532, 534) zum Analysieren von mindestens einer dieser Reihen von Werten verfügt, wobei mindestens einer der Bearbeitungsschritte aus der Gruppe ausgeführt wird, die folgendes umfaßt:

- Berechnung (532) von Mittelwerten und/oder Statistiken;

- Schätzung und/oder Vorhersage (534) zukünftiger Werte;

- Auslösen eines Alarmsignals (536) im Falle einer besorgniserregenden Entwicklung.

23. Verfahren zum Ermitteln des Arterienblutdruckes einer Person mit Hilfe eines kontinuierlich arbeitenden Blutdruckmeßgerätes, gemäß einem der Ansprüche 1 bis 22, dadurch gekennzeichnet, daß es eine Initialisierungsphase (101) und eine kontinuierliche Betriebsphase (102) umfaßt, wobei die Initialisierungsphase (101) darin besteht, daß mindestens eine Referenzinformation gespeichert wird, die für das Verhältnis zwischen mindestens einer absoluten Messung des Arterienblutdruckes, ausgeführt mit einem Referenzblutdruckmeßgerät, und mindestens einer Messung des für den mit dem kontinuierlich arbeitenden Blutdruckmeßgerätes gemessenen augenblicklichen Blutdruck repräsentativen Signals, repräsentativ ist, und wobei die kontinuierliche Betriebsphase (102) in der regelmäßigen Durchführung des Zyklus mit den folgenden Phasen besteht:

- Messung (1012) des für den augenblicklichen Blutdruck repräsentativen Signals;

- Ermittlung (1016) des Arterienblutdruckes der untersuchten Person als Funktion der Messung(en) und der Referenzinformation(en).

**24.** Verfahren gemäß Anspruch 23, dadurch gekennzeichnet, daß die Initialisierungsphase (101) die folgenden Schritte umfaßt:

- Messung (103) eines systolischen und eines diastolischen Referenzdruckes der untersuchten Person mit Hilfe des Referenzblutdruckmeßgerätes;

- Einführung und Speicherung (103) dieser systolischen und diastolischen Referenzdrücke in dem kontinuierlich arbeitenden Blutdruckmeßgerät;

- Messung (106) des für den augenblicklichen Blutdruck repräsentativen Signals über einen vorbestimmten Zeitraum, für mindestens zwei vorbestimmte Druckwerte dieses Blutdruckmeßgerätes auf der Haut der untersuchten Person;

- Ermittlung (107) der Amplitude des Signals, welches für jede der vorbestimmten Druckwerte vom Blutdruckmeßgerät gemessen wird;

- Berechnung (1011) einer mathematischen Funktion, die für die Amplitudenvariation des als Funktion des Druckes des Blutdruckmeßgerätes auf der Haut der untersuchten Person gemessenen Signals repräsentativ ist.

**25.** Verfahren gemäß Anspruch 23 und 24, dadurch gekennzeichnet, daß es einen Validierungsschritt für die Initialisierung umfaßt, der im folgenden besteht:

- Berechnung (105) eines ersten Referenzformkoeffizienten, ausgehend von den Referenzdrücken;

- Ermittlung (108) eines zweiten Referenzformkoeffizienten, gemessen auf der Grundlage des gemessenen Signals;

- Vergleich (109) des ersten und zweiten Referenzformkoeffizienten.

**26.** Verfahren gemäß einem der Ansprüche 23 bis 25, dadurch gekennzeichnet, daß der Schritt zur Ermittlung des Arterienblutdruckes die folgenden Schritte umfaßt:

- Ermittlung (1013) der Amplitude des gemessenen Signals:

- Messung (1012) des Druckwertes des Blutdruckmeßgerätes auf der Haut der untersuchten Person;

- Berechnung (1016) des Systolen- und des Diastolendruckes.

**27.** Verfahren gemäß Anspruch 26, dadurch gekennzeichnet, daß es einen Schritt zur Validierung des kontinuierlichen Betriebszyklus umfaßt, bestehend in:

- der Berechnung (105) eines ersten Referenzformkoeffizienten, ausgehend von den Referenzdruckwerten;

- der Ermittlung (1013) eines zweiten Referenzformkoeffizienten, ausgehend vom gemessenen Signal;

- dem Vergleich (1014) des ersten und des zweiten Referenzformkoeffizienten;

- der Wiederaufnahme der Initialisierungsphase und/oder der Berechnung dieser Funktion, wenn der erste und der zweite Formkoeffizient verschieden sind.

**28.** Verfahren gemäß einem der Ansprüche 26 und 27, dadurch gekennzeichnet, daß der Schritt (1016) zum Berechnen des Systolen- und des Diastolendruckes im Berechnen der folgenden Werte besteht:

$$SBP = (3 \cdot k - 1) \cdot Y/q$$

$$DBP = (3 \cdot k - 2) \cdot Y/q$$

wobei: SBP der Systolendruck;
DBP der Diastolendruck;
Y die Amplitude des gemessenen Signals;
k der gemessene Formkoeffizient;
q ein Eichkoeffizient für das Blutdruckmeßgerät sind.

Fig. 1

Fig. 2

Fig. 3

Fig. 4

Fig. 6

Fig. 5

Fig. 10

Fig. 7

Fig. 8

Fig. 9

Fig. 11

Fig. 12